# EUROPEAN PATENT APPLICATION

(11) **EP 3 900 635 A1**
(43) Date of publication of application: **27.10.2021**
(21) Application number: 20170975.5
(22) Date of filing: 23.04.2020
(51) Int. Cl.: A61B 8/08, A61B 8/06, A61B 8/00, G01S 7/52

(54) **VASCULAR SYSTEM VISUALIZATION**

(71) Applicant: Koninklijke Philips N.V., 5656 AG Eindhoven (NL)
(72) Inventor: MERT, Aydin, 5656 AE Eindhoven (NL); GROEN, Johanneke Gerrigje, 5656 AE Eindhoven (NL); VERBAKEL, Frank, 5656 AE Eindhoven (NL); IZAMIS, Maria-Louisa, 5656 AE Eindhoven (NL); NOTTEN, Marc Godfriedus Marie, 5656 AE Eindhoven (NL)
(74) Representative: Philips Intellectual Property & Standards

(57) **Abstract**

A method (100) for visualizing a section of a patient's vascular system (155) with an ultrasound image processing system (3) is disclosed. The method comprises receiving (103) ultrasound data generated by an ultrasound probe (10) being displaced across a part of the patient's anatomy (150) containing the section of the patient's vascular system, said ultrasound data comprising image data and Doppler data; generating (107) ultrasound images (170) from said image data, each ultrasound image corresponding to a subsection of the patient's vascular system having a determined location within the patient's vascular system and combining (109) said ultrasound images into a map (180) of said section of the patient's vascular system; processing (111) the received Doppler data to obtain blood flow characteristics for the section of the patient's vascular system; identifying (113) landmarks within the section of the patient's vascular system based on the obtained blood flow characteristics; annotating (115) said map with said landmark identifications (181, 183) and/or said blood flow characteristics; and generating (117) an output signal comprising said annotated map. Also disclosed are a computer program product and ultrasound image processing system for implementing this method.

## Description

### FIELD OF THE INVENTION

The present invention relates to a method for visualizing a section of a patient's vascular system with an ultrasound image processing system.

The present invention further relates to a method of tracking an invasive instrument comprising an ultrasound imaging arrangement through a patient's vascular system with an ultrasound image processing system.

The present invention further relates to computer program products for implementing such methods.

The present invention further relates to ultrasound image processing systems for implementing such methods.

### BACKGROUND OF THE INVENTION

In medical practice, vascular interventions are procedures in which a minimally invasive instrument such as a guidewire or catheter is inserted into the artery or a vein of a patient in order to correct an anomaly such as a stenosis within the patient's vascular system, for example by an angioplasty procedure in which a balloon is inserted into the patient's artery and inflated at the stenosis site such as to increase the effective diameter of the artery at that location, by implantation of a stent or graft, and so on. In such procedures, it is critically important that the minimally invasive instrument is guided to the appropriate location within the patient's vascular system. This therefore not only requires accurate positioning of the minimally invasive instrument relative to the site of the anomaly, but also navigation of the minimally invasive instrument through the patient's vascular system, e.g. to ensure the correct branch of a bifurcation in such a vascular system is taken.

In order to be able to perform such vascular interventions, it is important that the clinician has detailed knowledge of the section of the patient's vascular system containing the anomaly, such that the clinician knows the location of the anomaly within this section, as well as can determine the best access point into the section for the minimally invasive instrument. For this reason, the whole section of the patient's vascular system is typically imaged using X-ray imaging in preparation of the vascular intervention such that the clinician can mark the critical points within the section of the patient's vascular system directly onto the patient's skin, e.g. using a (marker) pen, thereby creating a mental map for the clinician to aid him or her during the actual minimally invasive procedure. Moreover, such markings can assist the clinician in guiding the minimally invasive instrument through the patient's vasculature during the actual procedure. Nevertheless, during such procedures, the clinician typically requires visualization of the (tip of the) minimally invasive instrument within the patient's vasculature, as well as visualization of landmarks such as the site(s) to be treated within the patient's vasculature in order to maximize the chance of success of the procedure.

At present, such vascular interventions are often performed under X-ray visualization. This is not ideal for a number of reasons. Firstly, the required X-ray equipment is costly. Secondly, such visualization exposes both the patient and clinician to radiation. This is particularly problematic for the clinician, as the clinician may have to perform many of such interventions (on different patients), in which in particular the hands of the clinician will be exposed to such radiation throughout the intervention as the clinician is guiding the minimally invasive instrument through the patient's vasculature and is performing the corrective procedure thereon. Such prolonged exposure to X-rays can lead to health issues such as accelerated skin ageing and mutations at the cellular level within the skin (e.g. the development of cancer). In addition, such X-ray imaging preferably is performed with the aid of a contrast agent administered to the patient in order to improve imaging contrast, but such contrast agents cannot be safely administered to patients with certain health problems, e.g. patients having kidney problems.
One possible solution to this problem would be to use ultrasound imaging during the procedure for such visualization problems. Ultrasound imaging has several advantages over X-ray imaging: not only is it harmless, but it can produce more detailed images, which can contribute to the success of the procedure. However, ultrasound images provide so much detail that interpretation of such images is extremely difficult to the untrained eye. In addition, correct (angular) positioning of the ultrasound probe relative to the patient's anatomy is far from trivial. This therefore typically requires the presence of an experienced sonographer working in tandem with the clinician during such procedures, in which the sonographer operates the ultrasound probe, interprets the ultrasound images and communicates the image results to the clinician. This is also costly in terms of requiring the presence of an additional highly trained medical practitioner (the sonographer). For these reasons, this so far has meant that X-ray visualization has not been widely replaced with ultrasound visualization during such vascular interventions.

US 2011/0306025 A1 discloses an apparatus and a method that reproduce a diagnostic or interventional procedure that is performed by medical personnel using ultrasound imaging. A simulator of ultrasound imaging is used for purposes such as training medical professionals, evaluating their competence in performing ultrasound-related procedures, and maintaining, refreshing, or updating those skills over time. This method may include using a plurality of 3-D image data sets from an image library to provide an operator with a complete visualization of an organ such as the human heart and its surrounding tissue by merging these image data sets. However, this approach is far from ideal for clinicians that simply need to be able to interpret ultrasound image maps without obtaining skills to acquire such maps, such as skilled vascular surgeons.

### SUMMARY OF THE INVENTION

The present invention seeks to provide a method for visualizing a section of a patient's vascular system with an ultrasound image processing system that allows for the interpretation of the visualization by medical practitioners with limited ultrasound imaging acquisition and/or interpretation skills.

The present invention further seeks to a method of tracking an invasive instrument comprising an ultrasound imaging arrangement through a patient's vascular system with an ultrasound image processing system that does not require interpretation by a sonographer.

The present invention further seeks to provide computer program products for implementing such methods.

The present invention further seeks to provide ultrasound image processing systems for implementing such methods.

According to an aspect, there is provided a method for visualizing a section of a patient's vascular system with an ultrasound image processing system, the method comprising receiving ultrasound data generated by an ultrasound probe being displaced across a part of the patient's anatomy containing the section of the patient's vascular system, said ultrasound data comprising image data and Doppler data; generating ultrasound images from said image data, each ultrasound image corresponding to a subsection of the patient's vascular system having a determined location within the patient's vascular system and combining said ultrasound images into a map of said section of the patient's vascular system; processing the received Doppler data to obtain blood flow characteristics for the section of the patient's vascular system; identifying landmarks within the section of the patient's vascular system based on the obtained blood flow characteristics; annotating said map with said landmark identifications and/or said blood flow characteristics; and generating an output signal comprising said annotated map.

The creation of a panoramic map of the section of interest of the patient's vascular system by combining individual ultrasound images into such a map and the subsequent automatic annotation of such a map through evaluation of ultrasound Doppler and image data within this section of the patient's vascular system enables the visualization of the information (through annotation) that is of critical importance to the clinician performing the minimally invasive procedure, such that this clinician can make clinical decisions, e.g. deciding an access point for the minimally invasive instrument, deciding on the size of instrument to use, and so on, based on this map. Furthermore, the clinician may rely on the panoramic map during navigation of the minimally invasive instrument, as the map in combination with the clinician's expertise may allow the clinician to manoeuvre the minimally invasive instrument through the patient's vasculature without the need for permanent visualization, thereby reducing or, in an ideal scenario, avoiding the exposure of both the patient and the clinician to harmful radiation, e.g. X-rays.

In an embodiment, the location of each subsection within the patient's vascular system may be determined by tracking the displacement of the ultrasound probe with respect to the patient's anatomy, such that each ultrasound image can be accurately associated with a particular location within the patient's vascular system.

In a preferred embodiment, the identification of landmarks within the section of the patient's vascular system based on the obtained blood flow characteristics comprises identifying a stenosis within the section of the patient's vascular system, such that the clinician is automatically presented with the location of the treatment area within the patient's vascular system without the need to evaluate the ultrasound image map. Identifying said stenosis preferably comprises identifying a location within the section of the patient's vascular system having an increased peak systolic blood flow velocity compared to a reference location within the section of the patient's vascular system. The reference location preferably is automatically identified by the ultrasound image processing system, for instance by selecting a location within the same blood vessel, e.g. artery, of the patient's vascular system comprising the stenosis having the lowest blood flow velocity. Identifying said stenosis may further comprise calculating an occlusion percentage of said stenosis using the formula (1 - L/R) ^{∗} 100, in which L and R are the area or diameter of the stenosis and a reference site within the section of the patient's vascular system respectively. This gives an accurate quantification of the size of the stenosis, which will aid the clinician in deciding the appropriate treatment for the stenosis.
The section of the patient's vascular system typically comprises a branched network of blood vessels (e.g. a tree structure). In an embodiment, identifying landmarks within the section of the patient's vascular system based on the obtained blood flow characteristics comprises segmenting said branched network of blood vessels based on the obtained blood flow characteristics to distinguish between arteries and veins within the said branched network; and optionally identifying bifurcations in said branched network based on said segmentation. Such landmark identification for example will assist the clinician in navigating the minimally invasive instrument through the patient's vasculature.

The method may further comprise determining dimensions of the section of the patient's vascular system from said ultrasound data, such as maximum diameter of the blood vessels within the section of the patient's vascular system. This for example may be used by the clinician to determine the appropriate size of the minimally invasive instrument(s), e.g. an angiogram balloon, to be used in the procedure. Alternatively, the method may include the automated recommendation of such appropriate size(s) of the minimally invasive instrument(s) by the ultrasound image processing system based on the determined dimensions of the imaged section of the patient's vascular system. The method may further comprise displaying the annotated map on a display device; receiving a location identification and an associated further annotation from a user interface;
augmenting the annotated map with the further annotation linked to the identified location; and generating a further output signal comprising the augmented annotated map. Such further annotations for example may be produced by the clinician, e.g. to label locations of interest on the annotated map, such as the access point into the patient's vascular system and locations for positioning ultrasound patches for imaging critical areas such as bifurcations and the treatment area during the procedure, on the annotated map, such that the clinician can rely to an even greater extent on the map during the minimally invasive procedure, and further reduce the need for harmful imaging techniques such as X-ray imaging during such a procedure.

In an embodiment, the method further comprises receiving further ultrasound data generated by a further ultrasound probe being displaced across said part of the patient's anatomy; tracking the displacement of the further ultrasound probe with respect to the augmented annotated map; generating further ultrasound images from said further ultrasound data; and displaying said further ultrasound images together with the augmented annotated map on said display device. In this manner, the clinician can compare the images generated with the further ultrasound probe with the annotated map, e.g. to identify a landmark such as the access point as previously labelled on the annotated map, such that the clinician can correctly insert the needle for providing access for the minimally invasive instrument into the patient's vascular system.

The method may further comprise comparing each further ultrasound image with a section of the augmented annotated map comprising the identified location upon receiving said further ultrasound image; and generating a notification signal upon recognizing the identified location within said further ultrasound image. Such a notification signal, e.g. an audible or visual signal, may assist the clinician in the correct positioning of the ultrasound probe over the landmark within the patient's vascular system. As previously explained, such a landmark may be a critical region during the procedure over which an ultrasound patch for imaging during the procedure is required, e.g. to detect the presence of the minimally invasive instrument in the critical region, to ensure that the minimally invasive instrument is correctly navigated into or through the critical region, or such a landmark may be the access point through which the minimally invasive instrument is to access the patient's vascular system.

During such an access procedure, the clinician may manoeuvre a hand-held ultrasound probe onto the access point, which access point may be determined by tracking the position of the ultrasound probe relative to the annotated map or the patient's anatomy (this in essence equates to the same thing, given that the annotated map is generated in reference to this anatomy), and/or by the physician visually establishing a match between the chosen access point on the panoramic map and the ultrasound image generated by the hand-held ultrasound probe. The ultrasound image(s) generated by the hand-held ultrasound probe are preferably generated in long-axis view such that the midline of the blood vessel is visualized in such an ultrasound image. The clinician for example may align a midline on the displayed image with a graphical representation of the ultrasound probe, such that clinician can be assured that a physical midline on the actual hand-held ultrasound probe is aligned with the blood vessel centre line, thereby maximizing the chances of successful insertion of a needle into this blood vessel at the chosen access point by alignment of the access needle with this physical midline.

According to another aspect, there is provided a method of tracking an invasive instrument comprising an ultrasound imaging arrangement through a patient's vascular system with an ultrasound image processing system, the method comprising providing an annotated map of the patient's vascular system as produced by the method of any of the herein described embodiments; receiving a stream of ultrasound data corresponding to a plurality of ultrasound images from the ultrasound imaging arrangement; comparing each of said ultrasound images with the annotated map; and determining a position of the invasive instrument within the patient's vascular system based on a match between said ultrasound image and a particular region of the annotated map. In this manner, the position of the (minimally) invasive instrument may be tracked on the offline annotated map

According to another aspect, there is provided a computer program product comprising a computer readable storage medium having computer readable program instructions embodied therewith for, when executed on a processor arrangement of an ultrasound image processing system, cause the processor arrangement to implement any of the embodiments of the herein described methods. Such a computer program product for example may be used to enhance the functionality of existing ultrasound image processing systems.

According to yet another aspect, there is provided an ultrasound image processing system comprising a processor arrangement; and any of the herein described computer program products, wherein the processor arrangement is adapted to execute the computer readable program instructions of said computer program product. Such an ultrasound image processing system can assist a clinician in performing vascular interventions with a reduced need for harmful imaging techniques such as X-ray managing, whilst not requiring the presence of a sonographer during such vascular interventions.

### BRIEF DESCRIPTION OF THE DRAWINGS

Embodiments of the invention are described in more detail and by way of non-limiting examples with reference to the accompanying drawings, wherein:
FIG. 1 schematically depicts an ultrasound imaging system according to an example embodiment;
FIG. 2 is a flowchart of a method according to an embodiment;
FIG. 3 schematically depicts an ultrasound data acquisition method for the method depicted in FIG. 2;
FIG. 4 schematically depicts the processing of ultrasound images in accordance with an embodiment;
FIG. 5 schematically depicts an annotated panoramic map of a patient's vasculature generated with the method depicted in FIG. 2;
FIG. 6 schematically depicts a vascular access procedure aided by the annotated panoramic map of FIG. 5;

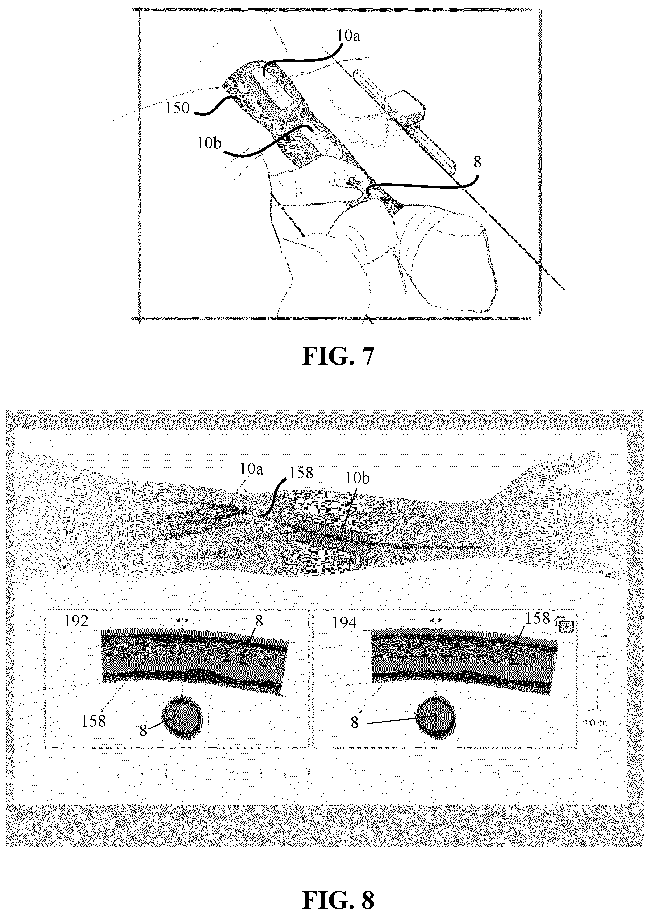

FIG. 7 schematically depicts a vascular navigation procedure aided by the annotated panoramic map of FIG. 5;
FIG. 8 schematically depicts the use of the annotated map of FIG. 5 in such a vascular navigation procedure according to an example embodiment; and
FIG. 9 is a flowchart of a method according to another embodiment.

### DETAILED DESCRIPTION OF THE EMBODIMENTS

It should be understood that the Figures are merely schematic and are not drawn to scale. It should also be understood that the same reference numerals are used throughout the Figures to indicate the same or similar parts.

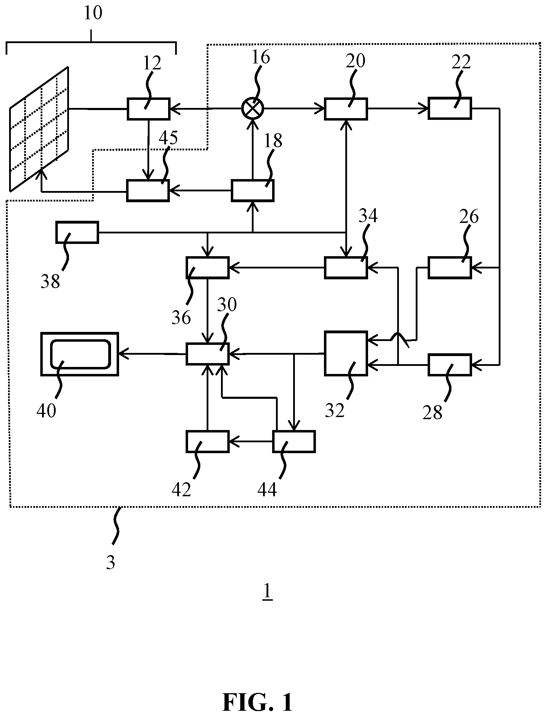

FIG. 1 schematically depicts an example embodiment of an ultrasound imaging system 1 including an ultrasound probe 10, e.g. an array of ultrasound transducer elements 66, which may be arranged in a one-dimensional or two-dimensional array of transducer elements, e.g. for the generation of 2-D and 3-D ultrasound images respectively. Any suitable type of ultrasound transducer elements 66 may be used for this purpose, e.g. piezoelectric transducer (PZT) elements, capacitive micro-machined ultrasound transducer (CMUT) elements, piezoelectric micro-machined transducer (PMUT) elements, and so on, although CMUT elements are particularly preferred, in particular over (PZT) elements due to their superior (adjustable) resonance frequency range, which make CMUT elements particularly suitable for patient monitoring purposes. As such transducer elements are well-known per se, they will not be explained in further detail for the sake of brevity only. Where reference is made to an ultrasound transducer element, it should be understood that this refers to a transducer unit addressable by a single control signal. This may be a single transducer cell or a cluster, e.g. tile, of transducer cells arranged to operate in unison, i.e. as a single element. The array of transducer elements may be arranged as a phased array to facilitate beam steering of an ultrasound beam generated with the ultrasound probe 10. Again, such beam steering is well-known per se and will not be explained in further detail for the sake of brevity only.

The ultrasound probe 10 may take any suitable shape, e.g. a hand-held probe, mounted probe, patch and so on. The ultrasound probe 10 typically is operable in a transmit mode in which ultrasound beams are generated and a receive mode in which the ultrasound probe 10 is operable to receive echo signals induced by the generated ultrasound beams within the body of the individual being imaged with the ultrasound probe 10. The ultrasound probe 10 typically is controlled by an ultrasound image processing system 3, and may be communicatively coupled thereto in any suitable manner, e.g. through a (coaxial) cable or the like. The ultrasound image processing system 3 may take any suitable shape, such as a user console, a portable electronic device such as a laptop, tablet computer or the like, or may at least in part be cloud-based, in that at least part of the processing of the data provided by the ultrasound probe 10 may be performed in the cloud, in which case such data may be provided to the cloud-based (part of the) ultrasound image processing system 3 over a network connection such as the Internet. As this is well-known per se, this will not be explained in further detail for the sake of brevity only.

The ultrasound probe 10 may be coupled to a microbeam former 12, which may be integrated in the ultrasound probe 10, which controls transmission and reception of signals by the ultrasound transducer elements (or clusters thereof) of the ultrasound probe 10. Microbeam formers are capable of at least partial beam forming of the signals received by groups or "patches" of transducer element tiles for instance as described in US patents US 5,997,479 (Savord et al.), US 6,013,032 (Savord), and US 6,623,432 (Powers et al.). The microbeam former 12 may be coupled by a probe cable 11, e.g. coaxial wire, to the ultrasound user console 3, e.g. a patient interface module or the like, comprising a transmit/receive (T/R) switch 16 which switches between transmission and reception modes and protects the main beam former 20 from high energy transmit signals when a microbeam former is not present or used and the ultrasound probe 10 is operated directly by the main system beam former 20. The transmission of ultrasonic beams from the ultrasound probe 10 under control of the microbeam former 12 may be directed by a transducer controller 18 coupled to the microbeam former by the T/R switch 16 and the main system beam former 20, which receives input from the user's operation of the user interface or control panel 38. One of the functions controlled by the transducer controller 18 is the direction in which beams are steered and focused. Beams may be steered straight ahead from (orthogonal to) the ultrasound probe 10, or at different angles for a wider field of view. The transducer controller 18 may be coupled to control the voltage source 45 for the ultrasound probe 10. For instance, the power supply 45 may set the DC and AC bias voltage(s) that are applied to CMUT cells in case of a CMUT probe 10, e.g. to operate the one or more CMUT cells of the CMUT elements in collapse mode, as is well-known per se.

The partially beam-formed signals produced by the microbeam former 12 may be forwarded to the main beam former 20 where partially beam-formed signals from individual patches of transducer elements are combined into a fully beam-formed signal. For example, the main beam former 20 may have 128 channels, each of which receives a partially beam-formed signal from a patch of dozens or hundreds of ultrasound transducer cells. In this way the signals received by thousands of transducer cells of a transducer array 10 can contribute efficiently to a single beam-formed signal.

The beam-formed signals are coupled to a signal processor 22, which may form part of the processor arrangement 50 of the ultrasound user console 3 by way of non-limiting example only. The signal processor 22 can process the received echo signals in various ways, such as bandpass filtering, decimation, I and Q component separation, and harmonic signal separation which acts to separate linear and nonlinear signals so as to enable the identification of nonlinear (higher harmonics of the fundamental frequency) echo signals returned from tissue and microbubbles.

The signal processor 22 optionally may perform additional signal enhancement such as speckle reduction, signal compounding, and noise elimination. The bandpass filter in the signal processor 22 may be a tracking filter, with its passband sliding from a higher frequency band to a lower frequency band as echo signals are received from increasing depths, thereby rejecting the noise at higher frequencies from greater depths where these frequencies are devoid of anatomical information.

The processed signals may be forwarded to a B-mode processor 26 and a Doppler processor 28, which processors also may form part of the processor arrangement 50. The B-mode processor 26 employs detection of an amplitude of the received ultrasound signal for the imaging of structures in the body such as the tissue of organs and vessels in the body. B-mode images of structure of the body may be formed in either the harmonic image mode or the fundamental image mode or a combination of both for instance as described in US Patents US 6,283,919 (Roundhill et al.) and US 6,458,083 (Jago et al.)

The Doppler processor 28 processes temporally distinct signals from tissue movement and blood flow for the detection of the motion of substances, such as the flow of blood cells in the image field. The Doppler processor typically includes a wall filter with parameters which may be set to pass and/or reject echoes returned from selected types of materials in the body. For instance, the wall filter can be set to have a passband characteristic which passes signal of relatively low amplitude from higher velocity materials while rejecting relatively strong signals from lower or zero velocity material.

This passband characteristic will pass signals from flowing blood while rejecting signals from nearby stationary or slowing moving objects such as the wall of the heart. An inverse characteristic would pass signals from moving tissue of the heart while rejecting blood flow signals for what is referred to as tissue Doppler imaging, detecting and depicting the motion of tissue. The Doppler processor may receive and process a sequence of temporally discrete echo signals from different points in an image field, the sequence of echoes from a particular point referred to as an ensemble. An ensemble of echoes received in rapid succession over a relatively short interval can be used to estimate the Doppler shift frequency of flowing blood, with the correspondence of the Doppler frequency to velocity indicating the blood flow velocity. An ensemble of echoes received over a longer period of time is used to estimate the velocity of slower flowing blood or slowly moving tissue.

The structural and motion signals produced by the B-mode (and Doppler) processor(s) are coupled to a scan converter 32 and a multiplanar reformatter 44, both which may also form part of the processor arrangement 50. The scan converter 32 arranges the echo signals in the spatial relationship from which they were received in a desired image format. For instance, the scan converter may arrange the echo signal into a two dimensional (2D) sector-shaped format, or a pyramidal three dimensional (3D) image.

The scan converter can overlay a B-mode structural image with colors corresponding to motion at points in the image field with their Doppler-estimated velocities to produce a color Doppler image which depicts the motion of tissue and blood flow in the image field. The multiplanar reformatter 44 will convert echoes which are received from points in a common plane in a volumetric region of the body into an ultrasonic image of that plane, for instance as described in US Patent US 6,443,896 (Detmer). A volume renderer 42, which also may form part of the processor arrangement 50, converts the echo signals of a 3D data set into a projected 3D image as viewed from a given reference point as described in US Pat. 6,530,885 (Entrekin et al.).

The 2D or 3D images are coupled from the scan converter 32, multiplanar reformatter 44, and volume renderer 42 to an image processor 30 forming part of the processor arrangement 50 for further enhancement, buffering and temporary storage for display on an image display device 40. In addition to being used for imaging, the blood flow values produced by the Doppler processor 28 and tissue structure information produced by the B-mode processor 26 are coupled to a quantification processor 34. The quantification processor produces measures of different flow conditions such as the volume rate of blood flow as well as structural measurements such as the sizes of organs and gestational age. The quantification processor may receive input from the user interface or control panel 38, such as the point in the anatomy of an image where a measurement is to be made.

Output data from the quantification processor is coupled to a graphics processor 36 for the reproduction of measurement graphics and values with the image on the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface or control panel 38, such as patient name. The user interface or control panel 38 may take any suitable shape, such as keyboard, touch screen, e.g. as part of the image display device 40, mouse, trackball and so on, or any combination of such user interface devices.

The user interface may also be coupled to the transmit controller 18 to control the generation of ultrasound signals from the ultrasound probe 10 and hence the images produced by the transducer array and the ultrasound system. The user interface may also be coupled to the multiplanar reformatter 44 for selection and control of the planes of multiple multiplanar reformatted (MPR) images which may be used to perform quantified measures in the image field of the MPR images.

As will be understood by the skilled person, the above embodiment of an ultrasound imaging system 1 is intended to give a non-limiting example of such an ultrasonic diagnostic imaging system. The skilled person will immediately realize that several variations in the architecture of the ultrasound imaging system 1 are feasible without departing from the teachings of the present invention. For instance, as also indicated in the above embodiment, the microbeam former 12 may be omitted, the ultrasound probe 10 may not have 3D imaging capabilities and so on. Other variations will be apparent to the skilled person. Specifically, where reference is made throughout this application to Doppler ultrasound imaging, it should be understood that this is to include power Doppler ultrasound imaging.

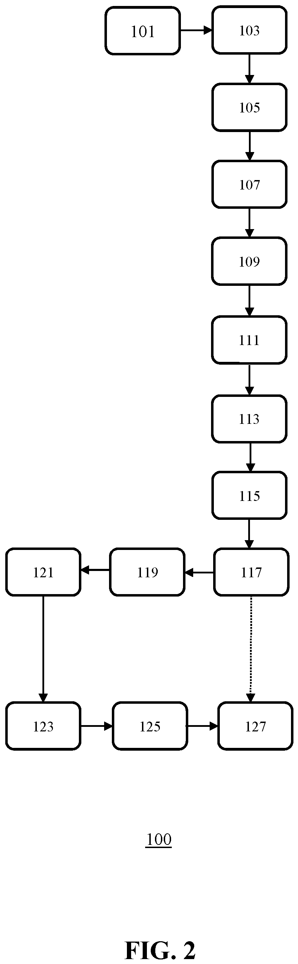

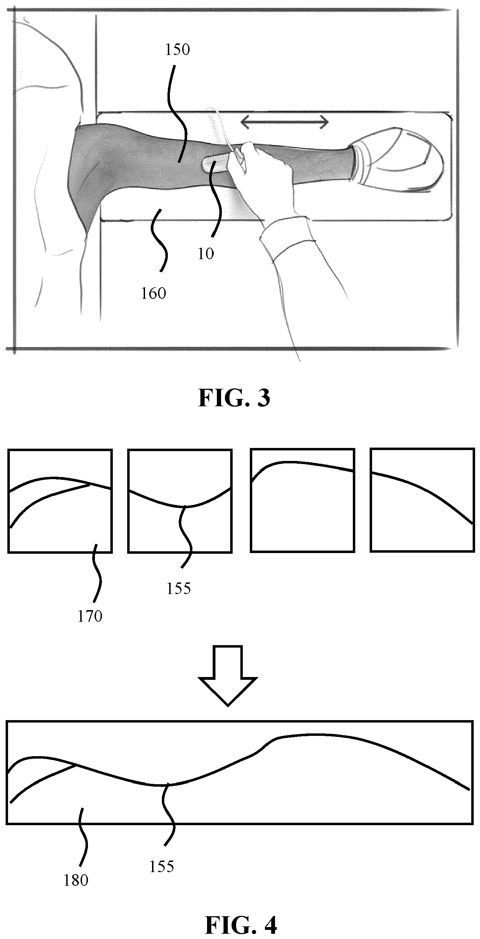

FIG. 2 is a flowchart of a method 100 according to an embodiment of the present invention as executed by the processor arrangement 50 of an ultrasound image processing system 3. The method 100 starts in 101, e.g. by an operator such as a clinician positioning an ultrasound probe 10 an anatomical portion of a patient such as an arm or a leg that contains a section of the patient's vascular system to be subjected to a vascular intervention, e.g. to treat a stenosis or the like. In operation 103, the operator moves the ultrasound probe 10 along the patient's anatomical feature 150 as schematically depicted in FIG. 3, in which this displacement is symbolized by the double arrow, thereby generating ultrasound data with the ultrasound probe 10, which ultrasound data typically comprises image data and Doppler data. The ultrasound probe 10 preferably captures such ultrasound data at different locations along the patient's anatomical feature 150, e.g. at regular intervals, i.e. the ultrasound probe 10 captures ultrasound image and (power) Doppler data of different parts of the vascular system within the patient's anatomical feature 150. The thus acquired ultrasound data is transmitted to the processor arrangement 50 for processing, as will be explained in more detail below. The ultrasound probe 10 is shown as a hand-held probe in FIG. 3 by way of non-limiting example only. It should be understood that any suitable ultrasound probe arrangement, e.g. an ultrasound probe 10 in a probe holder in which the ultrasound probe 10 can be (semi-)automatically translated along the anatomical feature 150, an (adhesive) ultrasound patch, and so on, is equally feasible.

At the same time, the position of the ultrasound probe 10 with respect to the patient's anatomical feature 150 during the displacement of the of the ultrasound probe 10 along the anatomical feature 150 of the patient is tracked by the processor arrangement 50 in operation 105, such that the position of the probe relative to the anatomical feature 150 is known at all times. In other words, for each ultrasound image generated from the ultrasound data, the position of the ultrasound probe 10 on the patient's anatomical feature 150 is known. This is important, as this means that during evaluation of the processed ultrasound data, as will be explained in more detail below, the clinician can relate this to actual locations on the patient's anatomical feature 150 with confidence. Such tracking may be done in any suitable manner. For instance, the ultrasound probe 10 may be displaced in a controlled manner relative to a defined starting point on the patient's anatomical feature 150, the patient's anatomical feature 150 may be affixed, e.g. strapped, in a reproducible manner to a support 160 such as a table or the like and a reference frame or coordinate system defined relative to the support 160 or the affixed anatomical feature 150 such that movement of the ultrasound probe 10 can be tracked within such a reference frame or coordinate system, e.g. using optical tracking of the displacement of the ultrasound probe 1-0 relative to the patient's anatomical feature 150 such as an arm or leg with camera-based systems. As such tracking is well-known per se, this will not be explained in further detail. Alternatively, operation 105 in which the ultrasound probe 10 is tracked as explained above may be replaced by the association of each ultrasound image 170 to a particular location within the patient's anatomical feature 150 using suitable algorithms. This for instance may be done after construction of the panoramic map 180, in which the overall shape of the patient's vascular system as captured in this map may be matched with an image of the patient's anatomical feature 150, such that individual locations within the map may be derived from this matching.

The processor arrangement 50 receives the ultrasound data from the ultrasound probe 10 in operation 107 and processes this data to extract a plurality of ultrasound images 170 and Doppler information from this ultrasound data, for example as explained in more detail in the detailed description of FIG. 1. Each ultrasound image 170 typically corresponds to a different subsection of the patient's vascular system having a defined location within the patient's vascular system. Such subsections may be spatially overlapping or may be spatially abutting subsections. In operation 109, the processor arrangement combines, e.g. stitches together, the ultrasound images 170 to form a panoramic map 180 of the section of the vascular system 155 within the patient's anatomical feature 150 as schematically depicted in FIG. 4, which map typically provides a full cross-sectional view of the patient's anatomical feature 150 and the vascular system 155 therein. Such a panoramic map 180 may be a 2-D map or may be a volumetric (3-D) map, as will be readily understood by the skilled person. Any suitable stitching algorithm may be used for this purpose. As such stitching algorithms are well-known per se, this will not be further explained for the sake of brevity only.

In operation 111, the processor arrangement 50 evaluates the ultrasound data corresponding to the different locations (vascular subsections) within the map 180, and in particular the (Power) Doppler data to obtain blood flow characteristics for the section of the patient's vascular system 155 within anatomical feature 150. Such evaluation is well-known per se and therefore not further explained for the sake of brevity only. In operation 113, the processor arrangement 150 evaluates the obtained blood flow characteristics to identify landmarks within the section of the patient's vascular system 155. Such landmarks may include any information relating to the patient's vascular system of use to the clinician that can be derived from the ultrasound data, such as by way of non-limiting example: calcification location information, plaque location information, stenosis location information, dissection location information, bifurcation location information, blood flow information at defined locations, blood vessel characterization information, vascular dimensions information, and so on. As will be understood by the skilled person, this information may be derived from the ultrasound image data, e.g. using image recognition algorithms, from the Doppler data, or from a combination of both the ultrasound image data and Doppler data.

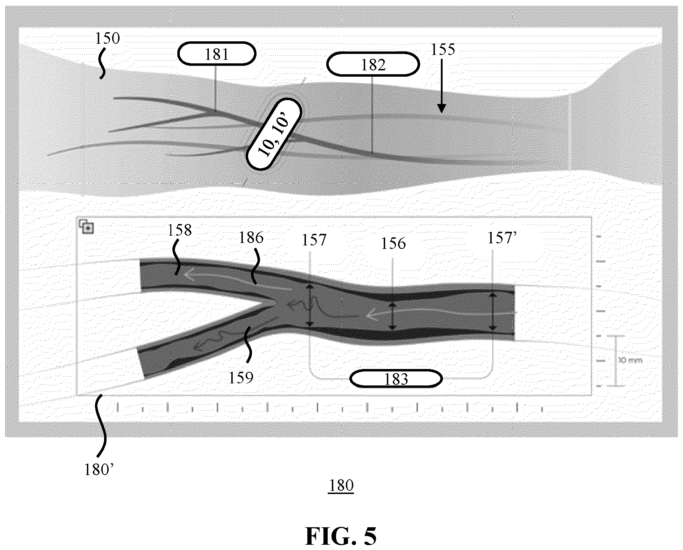

In a preferred embodiment, such landmark identification includes the identification of a stenosis 156 within the section of the vascular system 155 of the patient. Such identification clearly highlights the treatment area(s) to the clinician without the need for the clinician to interpret the ultrasound images in order to perform such identification. This is further explained with the aid of FIG. 5, which schematically depicts the panoramic map 180 including an inset 180', e.g. a magnification of a part of the map 180 selected by a user with the user interface 38 during displaying of the map 180 on the display device 40. In this preferred embodiment, the processor arrangement 50 may identify the stenosis 156 based on an increased peak systolic blood velocity at the location of the stenosis 156 compared to the (lowest) peak systolic blood velocity at adjacent arterial locations 157 and 157', i.e. upstream and downstream from the stenosis 156. This can be understood by the fact that the stenosis 156 reduces the effective diameter of the artery 158 at its location, thus causing the increased peak systolic blood velocity compared to the adjacent arterial locations 157, 157' where the effective diameter of the artery 158 is larger, which translates into a lower peak systolic blood velocity compared to the peak systolic blood velocity at the location of the stenosis 156.

In a further refinement, the size of the stenosis 156, i.e. of the stenotic occlusion may be quantified by determination of the effective arterial diameter L at the stenotic occlusion site and the effective arterial diameter R at an arterial reference location. The arterial reference location for example may be chosen by the processor arrangement 150 based on the peak systolic blood velocity, e.g. the location having the lowest peak systolic blood velocity (and therefore the highest effective arterial diameter) such as location 157 or 157'. The arterial diameters may be derived from the ultrasound imaging data, e.g. by determining the arterial diameter from a cross-sectional view of the artery 158 with the processor arrangement 50, e.g. from the panoramic map 180 or from the ultrasound image 170 corresponding to that particular location within the panoramic map 180, e.g. based on image recognition of the inner and/or outer vessel wall, of tissue or material around the vessel, and so on. Upon determination of the effective diameters R and L, the size S of stenosis 156 in the artery 158 may be expressed as a percentage by S (%) = (1-L/R) ^{∗} 100. It is noted that the landmark identification including the stenosis identification (and quantification) may be done at any suitable point in time, e.g. during generation of the panoramic map 180, or at a later point in time, e.g. on a different (computer) system than the ultrasound image processing system 3.

The processor arrangement 50 may further evaluate the blood flow characteristics in operation 113 for the purpose of deconstructing or segmenting the branched vessel network or vessel tree within the section of the vascular system 155 of the patient, for example for the purpose of distinguishing between arteries and veins, e.g. based on analysis of the pulsability of the blood vessel and/or blood flow profile through the vessel, and to identify bifurcations in the branched vessel network. Such information may be relevant to the clinician when navigating the minimally invasive instrument through the section of the vascular system 155 of the patient in order to reach the stenosis 156, as the identification of such bifurcations and blood vessel types can aid the clinician in identifying general directions and critical areas within the section of the vascular system 155 of the patient for navigation purposes.

In operation 115, the processor arrangement 50 annotates the panoramic map 180 with the derive landmark identifications and/or blood flow characteristics. Such annotations may take any suitable shape or form, and are typically presented in a legible format for the clinician, e.g. a label 181 or the like identifying a landmark such as a stenosis 156 or bifurcation within the section of the vascular system 155 of the patient, with the label 181 containing landmark identification information, e.g. "stenosis", "stenosis with occlusion percentage of 50%", "bifurcation", "maximum arterial diameter = 10 mm" and so on. The processor arrangement may further annotate the panoramic map 180 with blood flow rates, e.g. by annotating locations within blood vessels with the blood flow rate measured at that location so on. In this manner, the panoramic map 180 becomes easy to interpret by clinicians that are not ultrasound specialists, such as vascular surgeons. Advantageously, the processor arrangement 50 may further include an annotation 183 comprising a recommended balloon size for the vascular intervention based on the determined maximum diameter of the artery 158 as previously explained.

The processor arrangement 50, upon annotating the panoramic map 180 in this manner, proceeds to operation 117 in which it generates an output signal comprising the annotated map 180 (in digital form). The data pertaining to the annotated map 180 contained in this output signal may be stored on a data storage device, e.g. a hard drive, optical disk, network storage, cloud storage and so on, for retrieval at a later date, e.g. when commencing the vascular intervention on the patient. The method 100 may now terminate in operation 127.

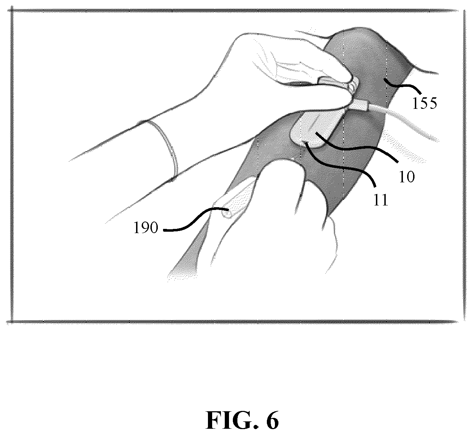

In an optional alternative embodiment, following operation 117, the output signal is used to control the display device 40 to display the annotated map 180 in operation 119. The displayed annotated map 180 may be further annotated by the clinician, e.g. using the user interface 38, thereby causing the processor arrangement to receive the further annotations 182 in operation 121, augment the annotated map with the further annotations in operation 123 and generate a further output signal containing the augmented annotated map in operation 125 prior to the method 100 terminating in operation 127. Such user-defined annotations may for instance comprise information that the clinician wishes to add to the annotated map 180 in preparation for the vascular intervention, such as the chosen access point location into the artery 158 containing the stenosis 156 in order to allow the clinician to find this access point in a straightforward manner when attempting to insert a needle 190 or the like into the patient's vascular system 155 at the intended location on the patient's anatomical feature 150, as schematically depicted in FIG. 6.

For example, during such an access procedure, the clinician may use a further ultrasound probe 10', which preferably is tracked within the same reference frame as the ultrasound probe 10, such that the location of the further ultrasound probe 10' can be superimposed on the (augmented) annotated map 180, e.g. when displayed on the display device 40, as schematically depicted in FIG. 5. This allows the clinician to direct the further ultrasound probe 10' to the correct location on the patient's anatomical feature 150 with the aid of the (augmented) annotated panoramic map 180. When this location is reached with the further ultrasound probe 10', i.e. the location of the further ultrasound probe 10' corresponds to the location within the annotated map 180 highlighting the access point into the patient's vascular system, the processor arrangement 50 may generate a notification signal causing the generation of a visual notification, e.g. on the display device 40 or on the further ultrasound probe 10', an audible notification, e.g. with a loudspeaker (not shown) of the ultrasound image processing system 3, and so on, to inform the clinician that the selected access point has been reached. At this point, it is noted that the further ultrasound probe 10'may be the same probe as the ultrasound probe 10, or may be a different probe.

In a further refinement, the processor arrangement 50 may compare the further ultrasound image(s) generated with the further ultrasound probe 10' with the access point location on the panoramic map 180 (or the corresponding ultrasound image 170) to determine a match between a further ultrasound image and the ultrasound image corresponding to the access point location, with such a match being indicative of the further ultrasound probe 10' having reached the access point location. At this point, the processor arrangement 50 may generate the aforementioned notification signal to notify the clinician that the further ultrasound probe 10' is in position over the access point location.

It is noted that in this embodiment, tracking of the further ultrasound probe 10' is not strictly necessary. Alternatively, if the further ultrasound probe 10' is tracked in a reference frame different to that of the ultrasound probe 10, an offset between the respective reference frames may be determined by matching a further ultrasound image with an ultrasound image corresponding to a chosen location within the reference frame of the ultrasound probe 10, as will be readily understood by the skilled person. Moreover, it should be understood that the further ultrasound image may be an image volume, e.g. constructed from a sequence of ultrasound images, as this may further facilitate the matching of the further ultrasound image to the annotated panoramic map 180.

The clinician may align a centered marker 11 on the further ultrasound probe 10' with a center line of the blood vessel to be accessed, e.g. artery 158, as depicted in the annotated panoramic map 180 such that the clinician can insert the needle 190 into the center of this blood vessel by aligning the needle 190 with the central marker 11. The access angle of the needle 190 into the blood vessel may be determined in a traditional manner, e.g. by displaying the further ultrasound image at the access point location in a long-axis view on the display device 40 together with the annotated panoramic map 180, e.g. in a split screen mode, during needle penetration.

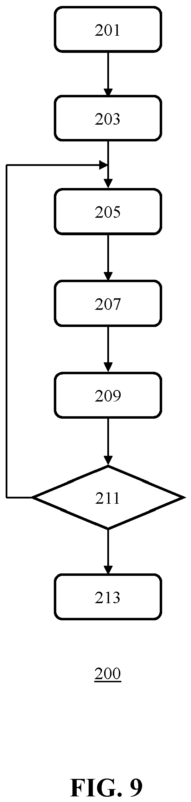

Following access, the clinician may insert a minimally invasive instrument such as a guide wire into the patient's vascular system 155 through the access point, and consult the annotated map 180, e.g. as displayed on the display device 40, to guide the minimally invasive instrument to the treatment area, e.g. the stenosis 156 without the need for a sonographer. Moreover, the clinician can use both hands for the navigation of the minimally invasive device 8, as there is no need to hold an ultrasound probe at the same time. Although the clinician may not have real-time visualization of the minimally invasive instrument within the patient's vascular system 155 using the annotated panoramic map 180 of the patient's vascular system 155, this map nevertheless will allow the clinician to navigate large parts of the patient's vascular system 155 without the need for such instrument imaging using his or her experience. Of course, the navigational route as determined on the annotated panoramic map 180 may contain some critical areas, e.g. bottlenecks such as malformations or bifurcations, the treatment area, and so on, in which live imaging of the minimally invasive instrument is desirable, e.g. to avoid incorrect navigation of the minimally invasive instrument. To this end, when further annotating the annotated panoramic map 180 as previously explained, the clinician may pinpoint (annotate) such critical locations on the annotated panoramic map 180, such that one or more additional ultrasound patches 10a, 10b can be positioned on the patient's anatomical feature 150 corresponding to the pinpointed critical locations prior to commencing the navigation of the minimally invasive instrument 8, as schematically depicted in FIG. 9. The ultrasound patches 10a, 10b may be affixed to these locations in any suitable manner, e.g. using an adhesive such as double-sided tape or the like.

As schematically depicted in FIG. 10, the ultrasound images 192, 194 produced by the ultrasound patches 10a, 10b respectively may be shown on the display screen 40 together the annotated panoramic map 180, e.g. by showing a long-axis and short-axis view produced by each ultrasound patch 10a, 10b, such that the location of the minimally invasive instrument 8 relative to the cross-section of the artery 158 can be clearly identified in short-axis view, whilst the long axis view shows the progress of the minimally invasive instrument 8 through the artery 158 at these critical locations. In this way, the clinician can utilize real-time visualization of the minimally invasive instrument 8, thereby further reducing the risk of navigation errors and increasing the visualization capability at the treatment site, e.g. at stenosis 156, which aids the efficiency and effectiveness of the treatment of the anomaly. To further assist the clinician in understanding where in the patient's vascular system 155 these critical points are visualized, a graphical representation of the actual positions of the ultrasound patches 10a, 10b may be visualized, e.g. superimposed, on the annotated panoramic map 180, as shown in FIG. 10. Moreover, such ultrasound patches 10a, 10b may provide real-time vessel information, such as blood flow velocities, vessel diameter, stenosis information, and so on, which may be presented in any suitable manner, e.g. as annotations in the ultrasound images produced by the ultrasound patches 10a, 10b.

In a particular embodiment, the minimally invasive instrument 8 may be equipped with ultrasound imaging capability. For example, the minimally invasive instrument 8 may be an intravascular ultrasound (IVUS) catheter. In such a scenario, the position of the (tip of the) minimally invasive instrument 8 may be visualized in real-time within the annotated panoramic map 180. This is explained in more detail with the aid of FIG. 11, which depicts a flowchart of a method 200 of tracking the (minimally) invasive instrument 8 comprising an ultrasound imaging arrangement through the patient's vascular system 155 with the ultrasound image processing system 3. The method 200 starts in operation 201, e.g. by insertion of the (minimally) invasive instrument 8 into the patient's vascular system 155 as previously explained, after which in operation 203 the processor arrangement 50 retrieves the annotated map 180 of the patient's vascular system, which may include the individual ultrasound images 170 in addition thereto. In operation 205, the processor arrangement 50 receives a stream of ultrasound data corresponding to a plurality of ultrasound images from the ultrasound imaging arrangement of the (minimally) invasive instrument 8 and compares each of said ultrasound images with the annotated map 180 in operation 207 to determine a position of the (minimally) invasive instrument 8 within the patient's vascular system 155 based on a match between the ultrasound image and a particular region of the annotated map 180 such that the position of (the tip of) the (minimally) invasive instrument 8 can be shown on the annotated map 180 in this particular region in operation 209, e.g. by superimposing a graphical representation of the (the tip of) the (minimally) invasive instrument 8 on the annotated map 180.

As before, this may be displayed onto the display device 40 in combination with the ultrasound images generated by (minimally) invasive instrument 8 within the artery 158, such that the clinician is presented with a real-time location of the (minimally) invasive instrument 8 within the artery 158 and well as a real-time view of the artery geometry at that location. It is subsequently checked in operation 211 if such monitoring is to continue, e.g. because the vascular intervention has not yet been completed. If this is the case, the method 200 reverts back to operation 205; otherwise, the method 200 terminates in operation 213.

Of course, many variations to such a method 200 may be contemplated. For instance, matching the position of the minimally invasive instrument to a location on the panoramic map 180 may be at least partially based on measuring the length of guide wire inserted into the patient's anatomy, such that this measured length can be used to estimate the location of the tip of the guide wire on the panoramic map 180, assuming that the entry point of the invasive instrument into the patient's anatomy is identified on the panoramic map 180. This for instance may be achieved based on a known insertion speed of the minimally invasive instrument into the patient's anatomy and/or using visual (distance) markers on the invasive instrument from which the total insertion length can be derived. As will be understood by the skilled person, this may be used with or without the aforementioned ultrasound image matching; in other words, this approach may also be used for estimating the location of minimally invasive instruments without imaging capability within the patient's vascular system as mapped by the panoramic map 180.

At this point it is reiterated that the above described aspects and embodiments of the present invention seek to reduce the exposure of both patient and clinician to harmful X-rays whilst at the same time obviating the need for a sonographer to be present during vascular interventions performed on the patient. It is however noted for the avoidance of doubt that the present invention does not suggest that the use of X-ray imaging for such procedures can be avoided altogether under all circumstances. For instance, there are scenarios in which a (minimally) invasive instrument 8 has to be navigated through areas with high bone density, e.g. the pelvic area, in which case navigation through such areas cannot be satisfactorily performed using ultrasound imaging, in which case X-ray imaging for navigation through such areas may still be required. Also, a clinician may desire the use of such X-ray imaging during treatment and/or during arrival of the (minimally) invasive instrument 8 at the treatment site within the patient's vascular system 155. Notwithstanding this, the various aspects of the present invention ensure that even where such X-ray imaging is still deployed, exposure times can be significantly reduced by the provision and use of the annotated panoramic map 180, as the clinician will be able to perform most of the navigation (and subsequent treatment of the patient) without requiring the support of X-ray imaging.

It is furthermore noted that the annotations 181 generated by the processor arrangement 50 on the panoramic map 180 may be generated in accordance with default settings, or in accordance with settings specified by the clinician, e.g. through the user interface 38. For example, the clinician may select, e.g. through a selection menu or the like displayed on the display screen 40, which particular types of information, e.g. types of landmarks, flow rates and so on, should be identified by the processor arrangement 50 and annotated on the map 180. Alternatively or additionally, the processor arrangement 50 may add all types of annotations to the panoramic map 180, but the clinician may suppress or select the displaying of particular types of annotations on the map 180, e.g. through a selection menu or the like displayed on the display screen 40 using the user interface 38. In this manner, the clinician can select the annotation information that is relevant to a particular vascular intervention, or appropriate to the level of expertise of the clinician. Of course, many other variations to the tailoring of such annotation information will be immediately apparent to the skilled person.

According to an aspect of the present invention, a computer program product may be provided comprising a computer readable storage medium having computer readable program instructions (code) embodied therewith for, when executed on the processor arrangement 50 of the ultrasound image processing system 3, cause the processor arrangement 50 to implement any embodiment of the method 100 or 200.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. Such a system, apparatus or device may be accessible over any suitable network connection; for instance, the system, apparatus or device may be accessible over a network for retrieval of the computer readable program code over the network. Such a network may for instance be the Internet, a mobile communications network or the like. More specific examples (a non-exhaustive list) of the computer readable storage medium may include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present application, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out the methods of the present invention by execution on the processor arrangement may be written in any combination of one or more programming languages, including an object oriented programming language such as Java, Smalltalk, C++ or the like and conventional procedural programming languages, such as the "C" programming language or similar programming languages. The program code may execute entirely on the processor arrangement as a stand-alone software package, e.g. an app, or may be executed partly on the processor arrangement and partly on a remote server. In the latter scenario, the remote server may be connected to the ultrasound image processing device 10 through any type of network, including a local area network (LAN) or a wide area network (WAN), or the connection may be made to an external computer, e.g. through the Internet using an Internet Service Provider.

Aspects of the present invention are described above with reference to flowchart illustrations and/or block diagrams of methods, apparatus (systems) and computer program products according to embodiments of the invention. It will be understood that each block of the flowchart illustrations and/or block diagrams, and combinations of blocks in the flowchart illustrations and/or block diagrams, can be implemented by computer program instructions to be executed in whole or in part on the processor arrangement 50 of the ultrasound image processing system 3, such that the instructions create means for implementing the functions/acts specified in the flowchart and/or block diagram block or blocks. These computer program instructions may also be stored in a computer-readable medium that can direct the ultrasound image processing device 10 to function in a particular manner.

The computer program instructions may be loaded onto the processor arrangement 50 to cause a series of operational steps to be performed on the processor arrangement 50, to produce a computer-implemented process such that the instructions which execute on the processor arrangement 50 provide processes for implementing the functions/acts specified in the flowchart and/or block diagram block or blocks. The computer program product may form part of the ultrasound image processing system 3, e.g. may be installed on the ultrasound image processing system 3.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. A method (100) for visualizing a section of a patient's vascular system (155) with an ultrasound image processing system (3), the method comprising:
receiving (103) ultrasound data generated by an ultrasound probe (10) being displaced across a part of the patient's anatomy (150) containing the section of the patient's vascular system, said ultrasound data comprising image data and Doppler data;
generating (107) ultrasound images (170) from said image data, each ultrasound image corresponding to a subsection of the patient's vascular system having a determined location within the patient's vascular system and combining (109) said ultrasound images into a map (180) of said section of the patient's vascular system;
processing (111) the received Doppler data to obtain blood flow characteristics for the section of the patient's vascular system;
identifying (113) landmarks within the section of the patient's vascular system based on the obtained blood flow characteristics;
annotating (115) said map with said landmark identifications (181, 183) and/or said blood flow characteristics; and
generating (117) an output signal comprising said annotated map.

2. The method of claim 1, further comprises tracking (105) the displacement of the ultrasound probe (10) with respect to the patient's anatomy to determine said location.

3. The method (100) of claim 1 or 2, wherein identifying (113) landmarks within the section of the patient's vascular system (155) based on the obtained blood flow characteristics comprises identifying a stenosis (156) within the section of the patient's vascular system.

4. The method (100) of claim 3, wherein identifying said stenosis (156) comprises identifying a location within the section of the patient's vascular system (155) having an increased peak systolic blood flow velocity compared to a reference location within the section of the patient's vascular system.

5. The method (100) of claim 3 or 4, wherein identifying said stenosis (156) further comprises calculating an occlusion percentage of said stenosis using the formula (1 - L/R) ^{∗} 100, in which L and R are the area or diameter of the stenosis and a reference site within the section of the patient's vascular system respectively.

6. The method (100) of any of claims 1-5, wherein the section of the patient's vascular system (155) comprises a branched network of blood vessels, and wherein identifying (113) landmarks within the section of the patient's vascular system based on the obtained blood flow characteristics comprises segmenting said branched network of blood vessels based on the obtained blood flow characteristics to distinguish between arteries (158) and veins (159) within the branched network; and optionally identifying bifurcations in said branched network based on said segmentation.

7. The method (100) of any of claims 1-6, further comprising determining dimensions of the section of the patient's vascular system (155) from said ultrasound data.

8. The method (100) of any of claims 1-7, further comprising:
displaying (119) the annotated map on a display device;
receiving (121) a location identification and an associated further annotation (152) from a user interface;
augmenting (123) the annotated map with the further annotation linked to the identified location; and
generating (125) a further output signal comprising the augmented annotated map.

9. The method (100) of claim 8, further comprising:
receiving further ultrasound data generated by a further ultrasound probe (10, 10', 10a, 10b) being displaced across said part of the patient's anatomy;
tracking the displacement of the further ultrasound probe with respect to the augmented annotated map (180);
generating further ultrasound images (192, 194) from said further ultrasound data; and
displaying said further ultrasound images together with the augmented annotated map on said display device (40).

10. The method (100) of claim 9, further comprising:
comparing each further ultrasound image (192, 194) with a section of the augmented annotated map (180) comprising the identified location upon receiving said further ultrasound image; and
generating a notification signal upon recognizing the identified location within said further ultrasound image.

11. A method (200) of tracking an invasive instrument (8) comprising an ultrasound imaging arrangement through a patient's vascular system (155) with an ultrasound image processing system (3), the method comprising:
providing (203) an annotated map (180) of the patient's vascular system as produced by the method (100) of any of claims 1-10;
receiving (205) a stream of ultrasound data corresponding to a plurality of ultrasound images from the ultrasound imaging arrangement;
comparing (207) each of said ultrasound images with the annotated map; and
determining (209) a position of the invasive instrument within the patient's vascular system based on a match between said ultrasound image and a particular region of the annotated map.

12. A computer program product comprising a computer readable storage medium having computer readable program instructions embodied therewith for, when executed on a processor arrangement (50) of an ultrasound image processing system (3), cause the processor arrangement to implement the method (100) of any of claims 1-10.

13. A computer program product comprising a computer readable storage medium having computer readable program instructions embodied therewith for, when executed on a processor arrangement (50) of an ultrasound image processing system (3), cause the processor arrangement to implement the method (200) of claim 11.

14. An ultrasound image processing system (3) comprising a processor arrangement (50); and
the computer program product of claim 12, wherein the processor arrangement is adapted to execute the computer readable program instructions of said computer program product.

15. An ultrasound image processing system (3) comprising a processor arrangement (50); and
the computer program product of claim 13, wherein the processor arrangement is adapted to execute the computer readable program instructions of said computer program product.
